# EUROPEAN PATENT SPECIFICATION

(11) **EP 2 708 249 B1**
(45) Date of publication and mention of the grant of the patent: **20.07.2016**
(21) Application number: 12782987.7
(22) Date of filing: 23.04.2012
(51) Int. Cl.: A61M 1/14, A61M 1/36

(54) **DIALYSIS DEVICE**
DIALYSEVORRICHTUNG
DISPOSITIF DE DIALYSE

(30) Priority: 10.05.2011 JP 2011105545
(43) Date of publication of application: 19.03.2014
(73) Proprietor: Nipro Corporation, Osaka-shi, Osaka 531-8510 (JP); Shibuya Kogyo Co., Ltd., Ishikawa 920-8681 (JP)
(72) Inventor: SHINZATO Toru, Nagoya-shi Aichi 462-0841 (JP); UEDA Mitsutaka, Osaka-shi Osaka 531-8510 (JP); SAWADA Toshiharu, Kanazawa-shi Ishikawa 920-8681 (JP); OKEDA Shogo, Kanazawa-shi Ishikawa 920-8681 (JP)
(74) Representative: Oxley, Rachel Louise
(86) International application number: PCT/JP2012/060821
(87) International publication number: WO 2012/153623

(56) References cited:
- JP-A- 10 043 290
- JP-A- 2001 252 352
- JP-A- 2001 252 352
- JP-A- 2009 131 412
- JP-A- 2012 005 721
- US-A1- 2010 192 686

## Description

### Technical Field

The present invention relates to a dialysis apparatus, and more specifically relates to a dialysis apparatus that is favorable when blood in a blood circuit is returned to a patient.

### Background Art

Conventionally, dialytic treatment using dialysis apparatuses are publicly known, and systems for returning blood remaining in a blood circuit after a dialytic treatment to a patient by means of backfiltration is also publicly known (for example, Patent Document 1).

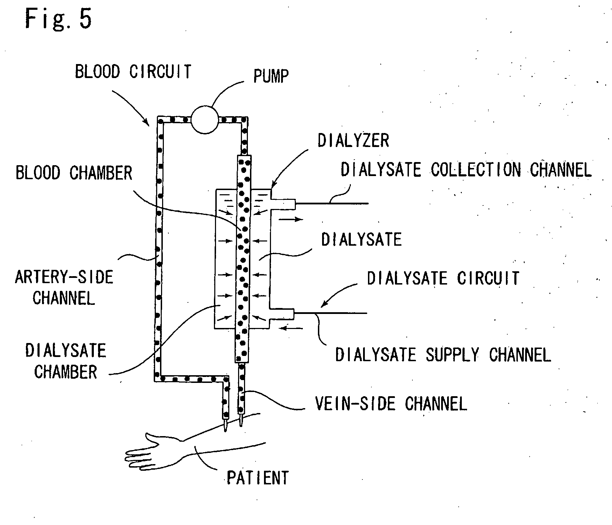

Here, as illustrated in Figure 5, "backfiltration" refers to, in a state in which the inside of a dialysate circuit is made to have a positive pressure relative to that of the inside of a blood circuit connected to a patient, a dialysate is made to flow from the dialysate circuit to a dialysate chamber in a dialyzer, whereby the dialysate is made to flow into a blood chamber from the dialysate chamber in the dialyzer and further flow into the blood circuit. As illustrated in Figure 5, in Patent Document 1 described above, backfiltration is performed to return blood remaining in the blood circuit to the patient. The blood return system according to Patent Document 1 has the advantage of being able to perform blood return using a relatively-inexpensive dialysate.

### Prior Art Documents

### Patent Documents

Patent Document 1: Japanese Patent No. 3858260

US 2010/192686 A1 discloses a method for returning blood to a patient using compressed air.

### Summary of the Invention

### Problems to be Solved by the Invention

However, systems of returning blood in a blood circuit to a patient by means of "backfiltration" processing as in Patent Document 1 described above have the following problem. As illustrated in Figure 5, a dialyzer includes a blood chamber, which is formed by an inner space of a multitude of hollow fiber membranes, and a dialysate chamber, which is provided lateral to the hollow fiber membranes and is an inner space of an outer covering case. When backfiltration is performed for returning blood, the entire dialysate chamber in the dialyzer is filled with a dialysate, and in such state, the dialysate that has penetrated the hollow fiber membranes flows into the blood chamber, and further flows into the blood circuit. Thus, when backfiltration is started, the dialysate flows into the entire blood chamber and mixed into blood, resulting in the problem of an increase in amount of blood diluted by the dialysate. Accordingly, there is the problem of a decrease in water removal effect using dialytic treatment.

Also, in dialytic treatment, a large amount of waste matter tends to accumulate and attach to a region around a part of the inside of the hollow fiber membranes (the inside of the blood chamber), the part being connected to an artery-side channel. Therefore, at the time of backfiltration processing after dialytic treatment, the dialysate flowing from the outside of the hollow fiber membranes (dialysate chamber) into the hollow fiber membranes (blood chamber) make the waste matter attaching to the inner surfaces of the hollow fiber membranes (inner surface of the blood chamber) be easily detached and returned to a patient together with blood, resulting in the problem of a decrease in effect of dialytic treatment.

### Means for Solving the Problems

In views of the aforementioned circumstances, the present invention according to claim 1 provides a dialysis apparatus comprising a dialyzer, an inside of which is separated by a semipermeable membrane into a blood chamber and a dialysate chamber, a blood circuit including an artery-side channel that has an end that includes a blood drawing port and another end connected to an inlet of the blood chamber in the dialyzer and a vein-side channel that has an end that includes a blood return port and another end connected to an outlet of the blood chamber, a dialysate circuit including a dialysate supply channel connected to an inlet of the dialysate chamber in the dialyzer and a dialysate collection channel connected to an outlet of the dialysate chamber, and backfiltration means for making a dialysate flow from the dialysate chamber into the blood chamber in the dialyzer as necessary, the backfiltration means being provided in the dialysate circuit, the dialys-ate being made to flow into the blood circuit via the dialyzer by the backfiltration means to return blood in the blood circuit, wherein the apparatus includes gas introduction means for introducing a gas into the dialysate chamber in the dialyzer, the gas is introduced into the dialysate chamber in the dialyzer by the gas introduction means, and in a state in which the dialysate remains in a region in the dialysate chamber other than a region in which the gas is introduced, the dialysate is made to flow into the blood circuit by the backfiltration means to perform blood return.

In the present invention according to claim 2, which is premised on the above configuration of claim 1, the dialyzer is vertically held so that the outlet of the dialysate chamber is positioned on an upper side, and the gas introduction means introduces the gas from the outlet of the dialysate chamber in the dialyzer to the dialysate chamber.

### Advantageous Effects of the Invention

According to the invention according to claim 1 stated above, the amount of dialysate flowing into the dialysate chamber in the dialyzer can be reduced relative to those of the conventional techniques, enabling reduction in amount of blood diluted by the dialysate, the blood being returned from the blood circuit to a patient.

Also, according to the invention according to claim 2, a gas is introduced from the outlet of the dialysate chamber on the upper side to the dialysate chamber, enabling suppression of gas bubble generation in the dialysate chamber compared to cases where a gas is introduced from the inlet on the lower side. Thus, a decrease in efficiency of blood return from the blood circuit to a patient due to attachment of gas bubbles to an outer surface of the blood chamber can be prevented.

### Brief Description of the Drawings

[Figure 1] Figure 1 is a perspective diagram illustrating an embodiment of the present invention.
[Figure 2] Figure 2 is a diagram illustrating a circuit configuration of the dialysis apparatus illustrated in Figure 1.
[Figure 3] Figure 3 includes cross-sectional views of a main part when blood return processing is performed by the dialysis apparatus illustrated in Figure 1: Figure 3(a) illustrates a state at the time of an end of a dialytic treatment; Figure 3(b) illustrates a state at the time of introduction of atmospheric air into a dialyzer; Figure 3(c) illustrates a preparatory step for blood return using backfiltration; and Figure 3(d) illustrates a state during backfiltration processing.
[Figure 4] Figure 4 is a cross-sectional view of a main part, which illustrates a process of processing using the dialysis apparatus illustrated in Figure 1: Figure 4(a) illustrates a state at the time of a dialytic treatment;
Figure 4(b) illustrates a preparatory step for blood return processing; and Figure 4(c) illustrates a state during blood return processing.
[Figure 5] Figure 5 is a cross-sectional view of a main part, which illustrates backfiltration processing using a conventional dialysis apparatus.

### Mode for Carrying Out the Invention

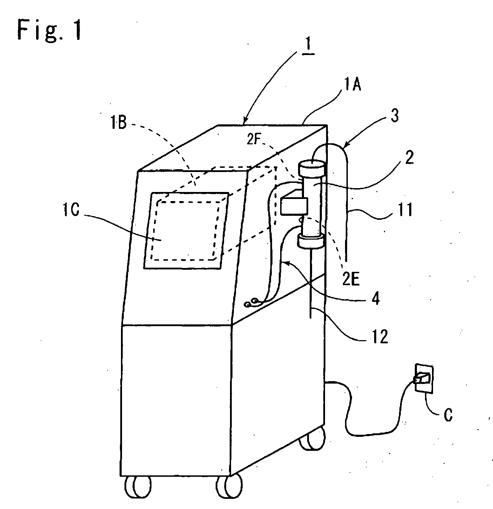

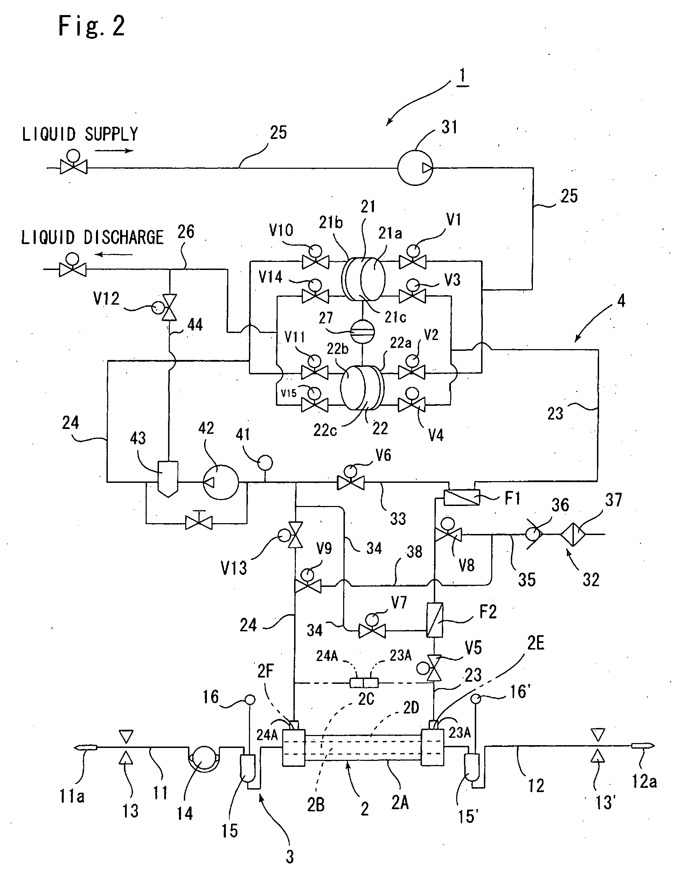

The present invention will be described below with reference to the illustrated embodiment. In Figures 1 and 2, a dialysis apparatus 1 starts operating upon supply of power from a power source such as an outlet C in a hospital, and the operation is controlled by control means 1B provided in a box-shaped main body portion 1A. The dialysis apparatus 1 according the present embodiment includes a cylindrical dialyzer 2 vertically held on a side surface of the main body portion 1A, a blood circuit 3 connected to the dialyzer 2, and a dialysate circuit 4 provided in the main body portion 1A and detachably connected to the dialyzer 2.

The control means 1B includes a screen display-type operation panel 1C, and on the screen, buttons, icons and messages necessary for operations are displayed, enabling operations of the apparatus and setting of various types of parameters.

The dialysis apparatus 1 according to the present embodiment is a dialysis apparatus for performing hemodialysis (HD) of one patient, and can perform priming immediately before a start to use the dialysis apparatus 1.

Also, in the dialysis apparatus 1, during a dialytic treatment, a pressure on the dialysate circuit 4 side can be higher than that on the blood circuit 3 side, enabling supply of a dialysate to a patient via the dialyzer 2 and the blood circuit 3. The pressure on the dialysate circuit 4 side being made to be higher to supply a dialysate to a patient via the dialyzer 2 and the blood circuit 3 as described above is conventionally and generally refers to "backfiltration". Also, in the present embodiment, "backfiltration" is performed by the dialysis apparatus 1 after a dialytic treatment to perform "blood return" processing for returning blood remaining in the blood circuit 3 to a patient, which will be described later.

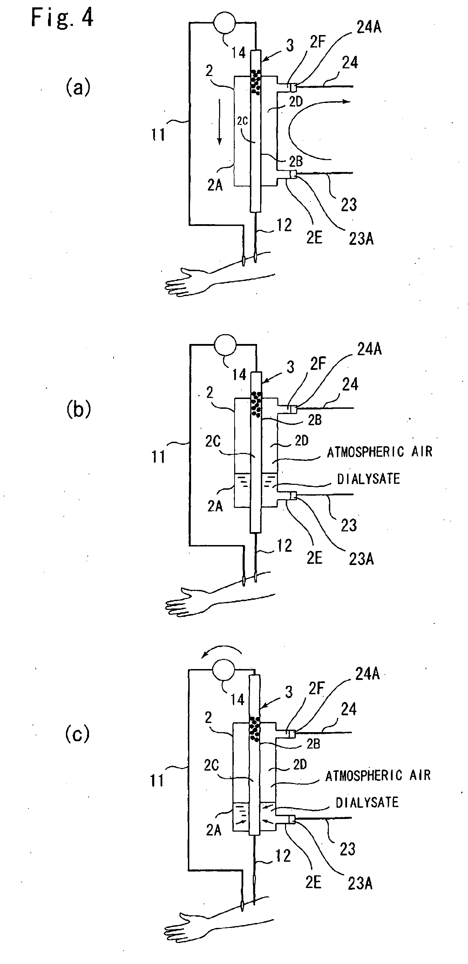

The dialyzer 2 has a configuration that is the same as those of the conventionally known ones, and the dialyzer 2 includes an outer covering case 2A including a cylindrical resin, and a multitude of hollow fiber membranes 2B provided inside the outer covering case 2A. Each of the hollow fiber membranes 2B is a filtration membrane (semipermeable membrane) and the inside of the hollow fiber membranes 2B provides a blood chamber 2C. The blood chamber 2C is in communication with the blood circuit 3, allowing blood to flow. The hollow fiber membranes 2B, as filtration membranes used in the dialyzer 2, have the property of letting liquid pass therethrough but not letting gas pass therethrough. Also, a space portion between an inner surface of the cylindrical outer covering case 2A and the blood chamber 2C (the respective hollow fiber membranes 2B) provides a dialysate chamber 2D that allows a dialysate to flow. At a lower portion of a side surface of the outer covering case 2A, an inlet 2E for introducing a dialysate to the dialysate chamber 2D is formed, and at an upper portion of the side surface of the outer covering case 2A, an outlet 2F for discharging the dialysate is formed. A dialysate supply channel 23 in the dialysate circuit 4, which will be described later, is connected to the inlet 2E, and a dialysate collection channel 24 in the dialysate circuit 4 is connected to the outlet 2F. Consequently, the inside of the dialysate chamber 2D is brought into communication with the dialysate circuit 4, whereby a dialysate flows in a direction opposite to that of blood (see Figure 4(a)).

In the present embodiment, the dialyzer 2 is vertically held on the side surface of the main body portion 1A so that the inlet 2E is positioned on the lower side and the outlet 2F is positioned on the upper side (see Figures 1 and 4).

As conventionally known, the blood circuit 3 is connected to a patient and blood is made to flow in the blood chamber 2C (hollow fiber membranes 2B) in the dialyzer 2 while a dialysate from the dialysate circuit 4 is made to flow in the dialysate chamber 2D in the dialyzer 2, enabling a dialytic treatment of the patient (see Figure 4(a)).

Next, the blood circuit 3 will be described. The blood circuit 3 includes an artery-side channel 11 for supplying blood to the blood chamber 2C in the dialyzer 2 and a vein-side channel 12 for returning blood from the blood chamber 2C in the dialyzer 2 to a patient when the artery-side channel 11 and the vein-side channel 12 are connected to blood vessels of the patient, and these channels each include a resin tube. As described above, the dialyzer 2 is vertically held on the main body portion 1A, and accordingly, the artery-side channel 11 is positioned on the upper side and the vein-side channel 12 is positioned on the lower side.

At an end of the artery-side channel 11, a puncture needle 11a to be inserted into a blood vessel of a patient is provided, and another end is connected to an inlet of the blood chamber 2C in the dialyzer 2. Also, in the artery-side channel 11, clamp means 13 as occlusion means for occluding the artery-side channel 11, a blood pump 14 that sends blood, and a drip chamber 15 are arranged in this order from the puncture needle 11a. The blood pump 14 is a roller pump that sends liquid by squeezing a tube, and operation of the blood pump 14 is controlled by the control means 1B, and the blood pump 14 enables blood to be sent from the patient to the blood chamber 2C in the dialyzer 2.

A manometer 16 is provided at, and non-illustrated liquid level adjustment means for adjusting a liquid level in the drip chamber 15 is connected to, the drip chamber 15 in the artery-side channel 11. The liquid level adjustment means is used when priming for filling the blood circuit 3 with a dialysate in advance before a start of a dialytic treatment, and is configured to adjust a liquid level in the drip chamber 15 to a predetermined level at that time. Also, during the dialytic treatment, the level of the liquid surface in the drip chamber 15 is maintained at a required level by the liquid level adjustment means.

An end of the vein-side channel 12 is connected to an outlet of the blood chamber 2C in the dialyzer 2, and at another end, a puncture needle 12a to be inserted into a blood vessel of the patient is provided. Also, in the vein-side channel 12, a drip chamber 15' and clamp means 13', which is occlusion means for occluding the vein-side channel 12 are provided in this order from the dialyzer 2, and at the drip chamber 15', a manometer 16' is provided.

Next, the dialysate circuit 4 will be described: however, a basic configuration of the dialysate circuit 4 is substantially the same as that of the conventional technique disclosed in, for example, Japanese Patent Laid-Open No. 10-43290. In other words, the dialysate circuit 4 includes a dialysate supply channel 23 that supplies fresh dialysate from a first dialysate chamber 21 or a second dialysate chamber 22 to the dialysate chamber 2D in the dialyzer 2, and a dialysate collection channel 24 that collects used dialysate that has passed through the dialysate chamber 2D in the dialyzer 2 into the first dialysate chamber 21 or the second dialysate chamber 22, and these channels each include a resin tube.

A liquid supply channel 25 that makes fresh dialysate supplied from a non-illustrated dialysate manufacturing apparatus and a liquid discharge channel 26 for discharging used dialysate are connected to the first and second dialysate chambers 21 and 22.

The first dialysate chamber 21 and the second dialysate chamber 22 have a same configuration, and the inside of each of the first dialysate chamber 21 and the second dialysate chamber 22 are separated by two diaphragms. In other words, the inside is separated into a supply chamber 21a or 22a for supplying fresh dialysate, a collection chamber 21b or 22b for collecting used dialysate, and an intermediate chamber 21c or 22c formed between the supply chamber 21a or 22a and the collection chamber 21b or 22b.

The inside of each of the intermediate chambers 21c and 22c are filled with a silicone oil, and the silicone oil in each of the intermediate chambers 21c and 22c is mutually sent between by the intermediate chamber 21c and the intermediate chamber 22 by an oil pump 27, whereby capacities of the intermediate chambers 21c and 22c are increased/decreased. In the present embodiment, the capacities of the intermediate chambers 21c and 22c in the respective chambers 21 and 22 are increased/decreased via the oil pump 27 to perform backfiltration during a dialytic treatment, enabling supply of a dialysate to a patient, and also to perform backfiltration after the dialytic treatment, enabling blood in the blood circuit 3 to be returned to the patient.

In the liquid supply channel 25, a liquid supply pump 31 that sends the dialysate is provided, and on the downstream side of the liquid supply pump 31, the liquid supply channel 25 is divided in two directions and connected to the supply chambers 21a and 22a in the first and second dialysate chambers 21 and 22. In the resulting divisional channels, liquid supply valves V1 and V2, which are opened/closed by the control means 1B, are provided, respectively.

An upstream part of the dialysate supply channel 23 is divided in two directions and connected to the supply chambers 21a and 22a in the first and second dialysate chambers 21 and 22, respectively, and an end portion on the downstream side is connected to the dialysate chamber 2D (inlet 2E) in the dialyzer 2 via a coupler 23A. The end portion of the dialysate supply channel 23 can be attached/detached to/from the inlet 2E via the coupler 23A. In the resulting divisional parts, supply valves V3 and V4, which are opened/closed by the control means 1B, are provided, respectively.

At positions in the dialysate supply channel 23 close to the dialyzer 2, a first dialysate filter F1 and a second dialysate filter F2, which remove harmful components in a dialysate, are arranged in series with a predetermined space therebetween, and furthermore, at a position between the second dialysate filter F2 and the dialyzer 2, a first on-off valve V5, which is opened/closed by the control means 1B, is provided. Opening means 32 for opening the dialysate supply channel 23 to atmospheric air as necessary is connected to a position in the dialysate supply channel 23 between the filters F1 and F2.

The inside of the first dialysate filter F1 is separated into the primary side (upstream chamber) and the secondary side (downstream chamber) by a semipermeable membrane, and when a dialysate passes from the primary side (upstream chamber) to the secondary side (downstream chamber) through the semipermeable membrane, harmful components are removed. A first bypass channel 33 that make the dialysate supply channel 23 and the dialysate collection channel 24 be in communication with each other is connected to the primary side (upstream chamber) of the first dialysate filter F1, and in the first bypass channel 33, a second on-off valve V6, which is opened/closed by the control means 1B, is provided.

The inside of the second dialysate filter F2 is also separated into the primary side (upstream chamber) and the secondary side (downstream chamber) by a semipermeable membrane, and when a dialysate passes from the primary side (upstream chamber) to the secondary side (downstream chamber) through the semipermeable membrane, harmful components are removed. A second bypass channel 34 that makes the dialysate supply channel 23 and the dialysate collection channel 24 be in communication with each other is connected to the primary side (upstream chamber) of the second dialysate filter F2, and in the second bypass channel 34, a third on-off valve V7, which is opened/closed by the control means 1B, is provided.

During a dialytic treatment, the first on-off valve V5 is opened while the second on-off valve V6 and the third on-off valve V7 are closed. On the other hand, for example, when an inadequacy in concentration of a dialysate is detected by a non-illustrated concentration sensor, the control means 1B closes the first on-off valve V5 and opens the second on-off valve V6. Consequently, a dialysate having the inadequate concentration can be sent to the dialysate collection channel 24 via the first bypass channel 33 without flowing to the dialyzer 2. As described above, in the present embodiment, the dialysate filters F1 and F2 are arranged in series in the dialysate supply channel 23, and the bypass channels 33 and 34 are arranged in parallel between the dialysate supply channel 23 and the dialysate collection channel 24.

In the present embodiment, the opening means 32 for detecting leaks in the dialysate filters F1 and F2 is provided. The opening means 32 includes an open channel 35 connected to a position between the dialysate filters F1 and F2 in the dialysate supply channel 23, a fourth on-off valve V8 provided in the open channel 35, the fourth on-off valve V8 being opened/closed by the control means 1B, a check valve 36 provided in the open channel 35, the check valve 36 blocking outflow of a dialysate, and an aseptic filter 37 that cleans atmospheric air that flows in.

The opening means 32 is used for inspection of leaks in the dialysate filters F1 and F2, and as necessary before or after a dialytic treatment, a leak inspection is performed using the opening means 32. More specifically, as necessary , the control means 1B closes the first on-off valve V5 and opens the second on-off valve V6, the third on-off valve V7 and the fourth on-off valve V8. Then, a flow of atmospheric air into the dialysate circuit 4 via the open channel 35 is allowed, enabling inspection of leaks in the dialysate filters F1 and F2 according to whether or not the atmospheric air flows from the secondary side (or the primary side) into the primary side (or the secondary side) of the dialysate filters F1 and F2. The atmospheric air flowing into the dialysate circuit 4 is cleaned by the aseptic filter 37 in the open channel 35, and an outflow of the dialysate in the dialysate circuit 4 is blocked by the check valve 36.

In the present embodiment, when blood in the blood circuit 3 is returned by means of backfiltration after a dialytic treatment, atmospheric air is introduced into the dialysate chamber 2D in the dialyzer 2, and at that time, the opening means 32 acts as gas introduction means. More specifically, a gas channel 38 that makes the open channel 35 and the dialysate collection channel 24 close to the dialyzer 2 be in communication with each other is provided, and in the gas channel 38, a fifth on-off valve V9, which is opened/closed by the control means 1B, is provided. When the fifth on-off valve V9 is opened by the control means 1B in a preparatory step for blood return processing after a dialytic treatment, which will be described in detail later, atmospheric air can be introduced into the dialysate chamber 2D in the dialyzer 2 via the open channel 35, the gas channel 38 and the dialysate collection channel 24 (see Figure 4(b)), and upon backfiltration being performed in such state, blood in the blood circuit 3 is returned to a patient (see Figure 4(c)).

Next, the dialysate collection channel 24 will be described; however, the dialysate collection channel 24 has a configuration that is the same as those of the conventional techniques. In other words, an end on the upstream side of the dialysate collection channel 24 is connected to the dialysate chamber 2D (outlet 2F) in the dialyzer 2 via a coupler 24A, and a downstream part is divided in two directions, and the resulting divisional parts are connected to the collection chambers 21b and 22b in the first and second dialysate chambers 21 and 22, respectively, and in the divisional parts, collection valves V10 and V11, which are opened/closed by the control means 1B, are provided, respectively. The end portion on the upstream side of the dialysate collection circuit 24 can be attached/detached to/from the outlet 2F via the coupler 24A, and as necessary, the coupler 24A can be detached from the outlet 2F.
Also, in the dialysate collection channel 24, a pressure sensor 41 that measures a pressure inside the dialysate circuit 4, a dialysate pump 42 that sends a dialysate, and a gas removal tank 43 that removes gas in the dialysate are provided in this order from the dialyzer 2 side, and between the gas removal tank 43 and the liquid discharge channel 26, a third bypass channel 44 is disposed. In the third bypass channel 44, a sixth on-off valve V12, which is opened/closed by the control means 1B, is provided. Also, at a position in a part connecting the second bypass channel 33 and the gas channel 38 in the dialysate collection channel 24, a seventh on-off valve V13 is provided. The seventh on-off valve V13 is opened/closed by the control means 1B as necessary.

During a dialytic treatment of a patient, the seventh on-off valve V13 is opened while the sixth on-off valve V12 is closed.

As described above, at the end portion of the dialysate supply channel 23, the coupler 23A is provided while at the end portion on the upstream side of the dialysate collection channel 24, also, the coupler 24A is provided. Thus, at the time of an inspection of leaks in the second dialysate filter F2, which will be described later, as indicated by imaginary lines in Figure 2, the couplers 23A and 24A are interconnected, enabling the channels 23 and 24 to be in communication with each other not via the dialyzer 2.

Next, an upstream part of the liquid discharge channel 26 is divided in two directions, and the resulting divisional parts are connected to the collection chambers 21b and 22b in the first and second dialysate chambers 21 and 22, respectively, and an end portion on the downstream side is connected to an non-illustrated liquid discharge tube installed in a medical institution, and in the divisional parts, liquid discharge valves V14 and V15, which are opened/closed by the control means 1B, are provided, respectively.

With the dialysis apparatus 1 having the above configuration, first, a dialytic treatment of a patient is performed, and also, as necessary during the dialytic treatment, backfiltration is performed to supply dialysate to the patient via the dialyzer 2 and the blood circuit 3. Then, upon end of the dialytic treatment of the patient, backfiltration is performed to make the dialysate flow in the dialysate chamber 2D in the dialyzer 2, whereby blood remaining in the blood circuit 3 is returned to the patient. A characteristic of the present embodiment lies in that when performing the blood return processing, a predetermined amount of atmospheric air is introduced into the dialysate chamber 2D in the dialyzer 2 in advance. Also, as necessary before or after the dialytic treatment, an inspection of leaks in the dialysate filters F1 and F2 is performed using the opening means 32.

A dialytic treatment, liquid supply processing using backfiltration and blood return processing using backfiltration after the dialytic treatment in the dialysis apparatus 1 will be described below.

First, a dialytic treatment of a patient is performed as follows. Before a start of a dialytic treatment, the puncture needles 11a and 12a are inserted into a patient to connect the blood circuit 3 to the patient, and subsequently, the blood pump 14 is activated to make blood flow in the blood circuit 3 (see Figures 2 and 4(a)).

In such state, the control means 1B opens the liquid supply valve V1 and the liquid discharge valve V14 and closes the supply valve V3 and the collection valve V10, and furthermore, the control means 1B activates the liquid supply pump 31 and opens the first on-off valve V5 and the seventh on-off valve V13. The other on-off valves V6 to V9 and V12 are closed.

Then, in the first dialysate chamber 21, since the liquid supply valve V1 and the liquid discharge valve V14 are opened, a dialysate from the liquid supply channel 25 flows into the supply chamber 21a, and in the collection chamber 21b, the diaphragms are pressed and the used dialysate that has previously been charged is discharged to the outside via the liquid discharge channel 26.

Meanwhile, in the second dialysate chamber 22, the control means 1B opens the supply valve V4 and the collection valve V11, and closes the liquid supply valve V2 and the liquid discharge valve V15. Then, used dialysate that has been sent by the dialysate pump 42 flows into the collection chamber 22b, and consequently, fresh dialysate is supplied from the supply chamber 22a into the dialysate chamber 2D in the dialyzer 2 via the dialysate supply channel 23.

Subsequently, the control means 1B alternately opens/closes the liquid supply valves V1 and V2, the supply valves V3 and V4, the collection valves V10 and V11, and the liquid discharge valves V14 and V15, whereby fresh dialysate from the first dialysate chamber 21 and the second dialysate chamber 22 is supplied to the dialysate chamber 2D in the dialyzer 2 via the dialysate supply channel 23, while used dialysate that has been passed through the chamber 2D in the dialyzer 2 is collected alternately by the first dialysate chamber 21 and the second dialysate chamber 22, and subsequently, is discharged to the outside of the dialysate circuit 4 via the liquid discharge channel 26. Such basic configuration of the dialysate circuit 4 is publicly known as in, e.g., Japanese Patent Laid-Open No. 10-43290 described above.

As described above, dialysate flows from the dialysate circuit 4 to the dialyzer 2, and blood flows into the blood chamber 2C in the dialyzer 2 via the blood circuit 3, whereby a dialytic treatment of a patient is performed.

Where it is necessary to supply a dialysate to a patient by means of "backfiltration" during a dialytic treatment, the following processing is performed. A necessary amount of silicone oil is supplied to the intermediate chamber 21c in the first dialysate chamber 21 in which dialysate subsequent to processing has been collected, by means of the oil pump 27. Consequently, the capacity of the collection chamber 21b becomes smaller than that of the supply chamber 21a in the first chamber 21, and as a result, an amount of dialysate corresponding to the difference in capacity therebetween is pressed into the blood circuit 3 via the blood chamber 2C in the dialyzer 2, that is, blood of the patient. In such a manner as described above, as necessary during a dialytic treatment, processing for dialysate supply using backfiltration is performed.

While a dialytic treatment of a patient is performed and dialysate supply using backfiltration is performed as necessary during the dialytic treatment as described above, it is necessary to inspect the filters F1 and F2 arranged in the dialysate supply channel 23 for leaks caused by damage. Therefore, the dialysis apparatus 1 according to the present embodiment, an inspection of the filters F1 and F2 for leaks is performed using the opening means 32 at a predetermined time before and after a dialytic treatment except during the dialytic treatment.

More specifically, in the present embodiment, first, a leak inspection of the first dialysate filter F1 is performed, and next, a leak inspection of the second dialysate filter F2 is performed. The leak inspection of the first dialysate filter F1 is performed as follows. In a state in which the liquid supply pump 31 is halted to stop supply of dialysate from the dialysate circuit 4, the control means 1B opens the second on-off valve V6, the fourth on-off valve V8 and the collection valve V11, and closes the supply valve V4, the first on-off valve V5 and the third on-off valve V7. In such state, a necessary amount of silicone oil is sent from the intermediate chamber 22c in the second dialysate chamber 22, which is the collection side, to the intermediate chamber 21c in the first dialysate chamber 21 by means of the oil pump 27. Along with that, the first bypass channel 33 and a part of the dialysate collection channel 24 on the downstream side relative to a position of connection with the first bypass channel 33 each have a negative pressure.

Thus, the dialysate in the secondary side (downstream chamber) of the first dialysate filter F1 penetrates the membrane that separates the inside and flows into the primary side (upstream chamber) of the first dialysate filter F1 and then is discharged to the collection chamber 22b via the first bypass channel 33 and the dialysate collection channel 24. Then, atmospheric air is introduced from the open channel 35 into the secondary side (downstream chamber) of the first dialysate filter F1 that has been emptied. When atmospheric air is introduced into the secondary side (downstream chamber) of the first dialysate filter F1 that has been emptied as described above, if there is a leak due to breakage of the membrane in the first dialysate filter F1, the pressure sensor 41 exhibits a value higher than a predetermined value. On the other hand, if there is neither breakage nor leak in the membrane in the first dialysate filter F1, the pressure sensor 41 exhibits the predetermined value. Accordingly, whether or not there is a leak in the first dialysate filter F1 can be detected. A leak inspection of the first dialysate filter F1 is first performed using the opening means 32 as described above.

Next, a leak inspection of the second dialysate filter F2 is performed as follows. In this case, first, the couplers 23A and 24A are detached from the dialyzer 2. and then interconnected to connect the channels 23 and 24 (see the imaginary lines in Figure 2). Subsequently, in a state in which the liquid supply pump 31 is halted to stop dialysate supply from the dialysate circuit 4, the control means 1B closes the supply valve V4, the second on-off valve V6 and the third on-off valve V7, and opens the first on-off valve V5, the fourth on-off valve V8, the collection valve V11 and the seventh on-off valve V13.

In such state, a necessary amount of silicone oil is sent from the intermediate chamber 22c in the second dialysate chamber 22, which is the collection side, to the intermediate chamber 21c in the first dialysate chamber 21 by means of the oil pump 27. Along with that, the inside of each of a part of the dialysate supply channel 23 downstream of the second dialysate filter F2, the couplers 23A and 24A and the dialysate collection channel 24 has a negative pressure. Accordingly, the dialysate in the secondary side (downstream chamber) of the second dialysate filter F2 is discharged to the collection chamber 22b through the channels 23 and 24 and the couplers 23A and 24A that each have the negative pressure, whereby the secondary side (downstream chamber) becomes empty. Then, atmospheric air is introduced to the primary side (upstream chamber) of the second dialysate filter F2 via the open channel 35 and a part of dialysate supply channel 23 at a position of connection with the open channel 35. The atmospheric air introduced to the primary side (upstream chamber) of the second dialysate filter F2 flows to the dialysate collection channel 24 through the second bypass channel 34, and is subjected to pressure detection by the pressure sensor 41.

When atmospheric air is introduced into the primary side (upstream chamber) of the second dialysate filter F2 as described above, if there is a leak due to breakage in the membrane in the second dialysate filter F2, the pressure sensor 41 exhibits a value higher than a predetermined value, while if there is no leak, the pressure sensor 41 exhibits the predetermined value. Accordingly, whether or not there is a leak in the second dialysate filter F2 can be detected.

As described above, in the present embodiment, atmospheric air is introduced to the respective dialysate filters F1 and F2 using the opening means 32 as necessary except during a dialytic treatment to inspect each of the dialysate filters F1 and F2 for leaks according to a difference in pressure values detected by the pressure sensor 41.

Furthermore, in the present embodiment, when the dialytic treatment has been performed and finished, blood of the patient remaining in the blood circuit 3 is returned to the patient using the above-described backfiltration. Here, in the present embodiment, as opposed to the conventional blood return processing, first, an amount of atmospheric air corresponding to around two-third of the inside of the dialysate chamber 2D in the dialyzer 2 is introduced into the dialysate chamber 2D as a preparatory step to maintain a state in which the dialysate is charged only in one-third of the inside of the dialysate chamber 2D, and in such state, backfiltration is performed for blood return.

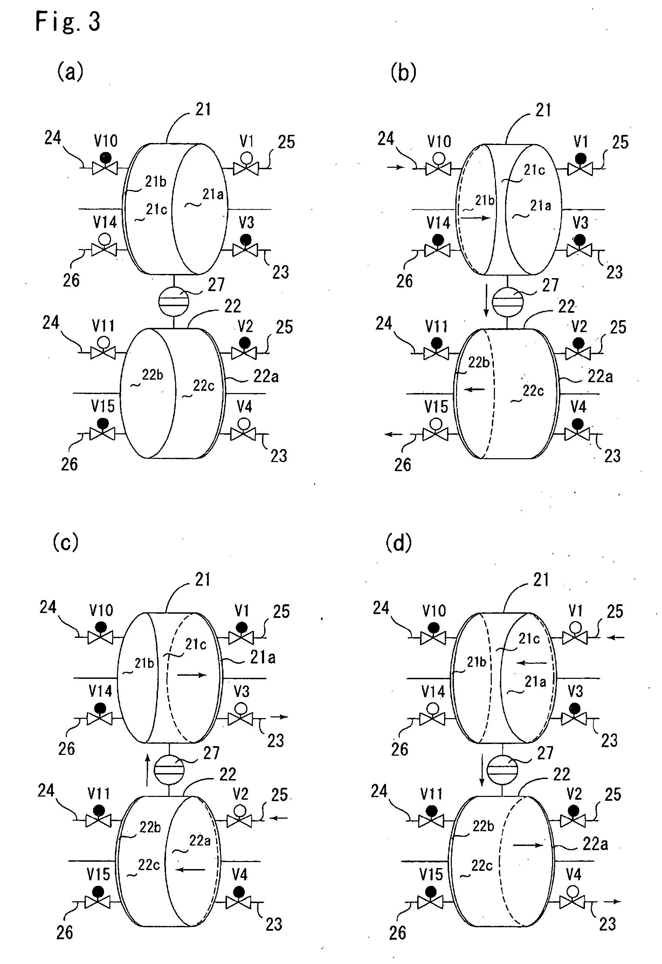

A process of introducing atmospheric air to around two-third of the inside of the dialysate chamber 2D will be described. At the time of the end of the above-described dialytic treatment, the first dialysate chamber 21 and the second dialysate chamber 22 are in the state illustrated in Figure 3(a). In other words, fresh dialysate introduced from the liquid supply channel 25 is in the supply chamber 21a in the first dialysate chamber 21 and used dialysate introduced from the dialysate collection channel 24 is in the collection chamber 22b in the second dialysate chamber 22.

In the state illustrated in Figure 3(a), the control means 1B closes the liquid supply valve V1, the supply valve V3 and the liquid discharge valve V14 on the first dialysate chamber 21 side, and closes the liquid supply valve V2, the supply valve V4 and the collection valve V11 on the second dialysate chamber 22 side (see Figure 3(b)). Next, the control means 1B opens the first on-off valve V5, the third on-off valve V7 and the fifth on-off valve V9, and closes the seventh on-off valve V13. Consequently, it becomes possible to introduce atmospheric air to the dialysate chamber 2D via the open channel 35, the gas channel 38, the dialysate collection channel 24 and the outlet 2F of the dialyzer 2.

Subsequently, a necessary amount of silicone oil is sent from the intermediate chamber 21c in the first dialysate chamber 21 to the intermediate chamber 22c in the second dialysate chamber 22 by means of the oil pump 27. Then, the dialysate in the dialysate chamber 2D in the dialyzer 2 passes through the dialysate supply channel 23, the second bypass channel 34 and a part of the dialysate collection channel 24 downstream of a position of connection with the second bypass channel 34 from the inlet 2E, and is collected into the collection chamber 21b via the collection valve V10. Along with that, the atmospheric air cleaned by the aseptic filter 37 and introduced to the open channel 35 and the gas channel 38 is introduced into the dialysate chamber 2D via a part of the dialysate collection channel 24 upstream of the fifth on-off valve V9 and the outlet 2F of the dialyzer 2. Concurrently with that, the dialysate in the collection chamber 22b in the second dialysate chamber 22 passes through the liquid discharge valve V15 and is discharged from the liquid discharge channel 26. Therefore, as the processing advances, the state of each of the first dialysate chamber 21 and the second dialysate chamber 22 changes from the state in Figure 3(a) to the state in Figure 3(b). The dashed arc lines in the chambers 21 and 22 in Figures 3(b) to 3(d) each indicate a position of the diaphragm before movement, and the arrow in the position adjacent to each dashed arc line indicates a direction of movement of the diaphragm.

Here, the control means 1B adjusts the amount of operation of the oil pump 27 so that around two-third of the inner space of the dialysate chamber 2D is replaced' with the atmospheric air. Therefore, a state in which the atmospheric air is introduced into an upper part that corresponds to around two-third of the inside of the dialysate chamber 2D in the dialyzer 2 and the dialysate remains only in a lower part that corresponds to one-third of the inside of the dialysate chamber 2D is maintained (see Figure 4(b)). In the present embodiment, the processing in the preparatory step for blood return is finished as described above.

Next, a blood return process using backfiltration will be described. From the state illustrated in Figure 3(b) in which the processing in the preparatory step has been finished as described above, the control means 1B opens the supply valve V3 in the first dialysate chamber 21 and closes the collection valve V10, and also opens the liquid supply valve V2 in the second dialysate chamber 22 and closes the liquid discharge valve V15 (see Figure 3(c)). Also, the control means 1B closes the third on-off valve V7, the fifth on-off valve V9 and the seventh on-off valve V13 in the dialysate circuit 4.

Then, a necessary amount of silicone oil is sent from the intermediate chamber 22c in the second dialysate chamber 22 to the intermediate chamber 21c in the first dialysate chamber 21 by means of the oil pump 27. Then, the dialysate contained in the supply chamber 21a in the first dialysate chamber 21 at the time of the end of the dialytic treatment passes through the supply valve V3 and then is introduced from the inlet 2E of the dialyzer 2 into the dialysate chamber 2D through the dialysate supply channel 23. The dialysate introduced into the dialysate chamber 2D is sent by pressure from the region corresponding to only one-third of the capacity of the dialysate chamber 2D in which the dialysate remains, to the blood circuit 3 via the blood chamber 2C (hollow fiber membrane 2B) (see Figure 4(c)). Also, fresh dialysate is introduced from the liquid supply channel 25 to the supply chamber 22a in the second dialysate chamber 22 through the liquid supply valve V2 (see Figure 3(c)). Accordingly, as the processing in this preliminary step advances, the state of each of the first dialysate chamber 21 and the second dialysate chamber 22 changes from the state in Figure 3(b) to the state in Figure 3(c).

In this case, the blood pump 14 rotates in a direction opposite to that at the time of the dialytic treatment at a rotation speed that provides a flow rate that is approximately a half of a flow rate of dialysate sent by pressure to the blood circuit 3 using backfiltration. Consequently, the dialysate sent by pressure to the blood chamber 2C is substantially equally distributed into the artery-side channel 11 and the vein-side channel 12.

If backfiltration of an amount of dialysate sufficient for blood return to the patient to the blood circuit 3 cannot be performed in the above-described processing in the preliminary step, backfiltration is performed again. In such case, from the state in Figure 3(c), the control means 1B opens the liquid supply valve V1 and the liquid discharge valve V14 for the first dialysate chamber 21 while closing the supply valve V3, and opens the supply valve V4 for the second dialysate chamber 22 while closing the liquid supply valve V2.

Then, a necessary amount of silicone oil is sent from the intermediate chamber 21c in the first dialysate chamber 21 to the intermediate chamber 22c in the second dialysate chamber 22 by means of the oil pump 27. Then, the dialysate is sent by pressure from the supply chamber 22a in the second dialysate chamber 22 in which fresh dialysate has been introduced in the previous backfiltration, to the blood circuit 3 through the supply valve V4 on a route similar to that of the previous backfiltration. Also, new dialysate is introduced from the liquid supply channel 25 to the supply chamber 21a in the first dialysate chamber 21 through the liquid supply valve V1. Accordingly, as the processing advances, each of the state of the first dialysate chamber 21 and the second dialysate chamber 22 changes from the state in Figure 3(c) to the state in Figure 3(d).

As described above, in the present embodiment, opening means 32 for a leak inspection of the dialysate filters F1 and F2 is used as gas introduction means to introduce atmospheric air to around two-third of the dialysate chamber 2D in the above-described preliminary step for blood return processing. In other words, the blood return processing is performed by performing backfiltration in a state in which the dialysate remains only in around one-third of the dialysate chamber 2D. As described above, in the present embodiment, compared to the conventional techniques in which backfiltration is performed with the inside of the entire dialysate chamber filled with a dialysate in order to perform blood return processing, a region in the blood chamber 2C into which dialysate flows can significantly be reduced. Thus, an amount of blood diluted by the dialysate can be reduced, the blood being returned from the blood circuit 3 to a patient, at the time of blood return processing.

Furthermore, the atmospheric air in the dialysate chamber 2D is introduced into a space portion that corresponds to an upper two-third portion of the inside of the dialysate chamber 2D, that is, the atmospheric air is introduced into the vicinity of the inlet side of the blood chamber 2C positioned on the upper side of the dialyzer 2. Thus, waste matter attached to the upper portion of the inside of the blood chamber 2C (hollow fiber membranes 2B) is not detached by dialysate flowing into the blood chamber 2C through the dialysate chamber 2D. Consequently, an amount of waste matter detached from the blood chamber 2C flowing into blood to be returned from the blood circuit 3 to the patient can be reduced, and thus, a decrease in effect of the dialytic treatment of the patient is prevented.

Furthermore, since the atmospheric air is introduced from the outlet 2F, which is on the upper side of the dialysate chamber 2D in the dialyzer 2, into the dialysate chamber 2D, air bubble generation in the dialysate chamber 2D can favorably be suppressed compared to cases where the atmospheric air is introduced from the inlet 2E, which is on the lower side, into the dialysate chamber 2D. Accordingly, a decrease in backfiltration efficiency, resulting from air bubbles generated in the dialysate chamber 2D attaching to outer surfaces of the hollow fiber membranes 2B can also be prevented.

Although in the above-described embodiment, "backfiltration" is performed using the capacity-variable dialysate chambers 21 and 22 each including a pair of diaphragms incorporated therein, "backfiltration" can be performed by reversely rotating a water removal pump provided in the dialysate circuit instead of such capacity-variable chambers. A dialysis apparatus including such water removal pump is publicly known as in, for example, Japanese Patent Laid-Open No. 2003-180823.

Also, although in the embodiment, the opening means 32 doubles as the gas supply means for supply a gas to the dialysate chamber 2D in the dialyzer 2, separate gas supply means may be provided without using the opening means 32 to supply a gas to the dialysate chamber 2D in the dialyzer 2 using the gas supply means at the time of blood return.

### Reference Signs List

- 1: dialysis apparatus
- 2: dialyzer
- 2C: blood chamber
- 2D: dialysate chamber
- 3: blood circuit
- 4: dialysate circuit
- 11: artery-side channel
- 12: vein-side channel
- 27: oil pump (backfiltration means)
- 32: opening means (gas introduction means)

## Claims

1. A dialysis apparatus (1) comprising:
a dialyzer (2), an inside of which is separated by a semipermeable membrane into a blood chamber (2C) and a dialysate chamber (2D);
a blood circuit (3) including an artery-side channel (11) that has an end that includes a blood drawing port and another end connected to an inlet of the blood chamber (2C) in the dialyzer (2) and a vein-side channel (12) that has an end that includes a blood return port and another end connected to an outlet of the blood chamber (2C);
a dialysate circuit (4) including a dialysate supply channel (23) connected to an inlet of the dialysate chamber (2D) in the dialyzer (2) and a dialysate collection channel (24) connected to an outlet of the dialysate chamber (2D); and
gas introduction means (32) for introducing a gas into the dialysate chamber (2D) in the dialyzer (2) in order to displace a dialysate to a region in the dialysate chamber (2D) other than a region containing the gas,
**characterized in that** the apparatus further comprises backfiltration means (27) provided in the dialysate circuit (4) for making the dialysate flow from the region containing the dialysate in the dialysate chamber (2D) into the blood circuit (3) via the blood chamber (2C) in the dialyzer (2),
and **in that** the gas introduction means (32) includes an open channel (35) that is provided in the dialysate supply channel (23) and allows air to be introduced to the dialysate supply channel (23), and a gas channel (38) that allows fluid communication between the open channel (35) and the dialysate collection channel (24).

2. The dialysis apparatus (1) according to claim 1, wherein the dialyzer (2) is vertically oriented so that the outlet of the dialysate chamber (2D) is positioned on an upper side, and the gas introduction means (32) is adapted to introduce the gas into the dialysate chamber (2D) in the dialyzer (2) at the outlet of the dialysate chamber (2D).

## Patentansprüche

1. Dialysegerät (1), umfassend:
einen Dialysator (2), dessen eine Innenseite durch eine semipermeable Membran in eine Blutkammer (2C) und eine Dialysatkammer (2D) unterteilt ist;
einen Blutkreislauf (3), der einen arterienseitigen Kanal (11), der ein Ende, das eine Blutabnahme-Öffnung umfasst, und ein anderes Ende, das mit einem Einlass der Blutkammer (2C) im Dialysator (2) verbunden ist, aufweist, und einen venenseitigen Kanal (12), der ein Ende, das eine Blutrückfluss-Öffnung umfasst, und ein anderes Ende, das mit einem Auslass der Blutkammer (2C) verbunden ist, aufweist, umfasst;
einen Dialysatkreislauf (4), der einen Dialysatzufuhrkanal (23), der mit einem Einlass der Dialysatkammer (2D) im Dialysator (2) verbunden ist, und einen Dialysatauffangkanal (24), der mit einem Auslass der Dialysatkammer (2D) verbunden ist, umfasst; und
ein Gaseinleitungsmittel (32) zur Einleitung eines Gases in die Dialysatkammer (2D) im Dialysator (2), um ein Dialysat in einen Bereich in der Dialysatkammer (2D), mit Ausnahme eines Bereichs, der das Gas enthält, zu verschieben;
**dadurch gekennzeichnet, dass** das Gerät ferner Rückfiltrierungsmittel (27) umfasst, die im Dialysatkreislauf (4) bereitgestellt sind, um das Dialysat zu veranlassen, von dem Bereich, der das Dialysat in der Dialysatkammer (2D) enthält, in den Blutkreislauf (3) über die Blutkammer (2C) im Dialysator (2) zu fließen;
und dadurch, dass das Gaseinleitungsmittel (32) einen offenen Kanal (35), der im Dialysatzufuhrkanal (23) bereitgestellt ist und ermöglicht, dass dem Dialysatzufuhrkanal (23) Luft zugeführt wird, und einen Gaskanal (38), der Fluidkommunikation zwischen dem offenen Kanal (35) und dem Dialysatauffangkanal (24) ermöglicht, umfasst.

2. Dialysegerät (1) gemäß Anspruch 1, worin der Dialysator (2) vertikal ausgerichtet ist, sodass der Auslass der Dialysatkammer (2D) auf einer oberen Seite angeordnet ist, und das Gaseinleitungsmittel (32) angepasst ist, das Gas in die Dialysatkammer (2D) im Dialysator (2) am Auslass der Dialysatkammer (2D) einzuleiten.

## Revendications

1. Dispositif de dialyse (1) comprenant :
un dialyseur (2) dont un intérieur est divisé par une membrane semi-perméable en une chambre de sang (2C) et une chambre de dialysat (2D),
un circuit de sang (3) comprenant un canal (11) du côté de l'artère qui comporte une extrémité qui comprend une connexion de tirage de sang et une autre extrémité connectée à une entrée de la chambre de sang (2C) dans le dialyseur (2) et un canal (12) du côté de la veine qui comporte une extrémité qui comprend une connexion de retour de sang et une autre extrémité connectée à une sortie de la chambre de sang (2C),
un circuit de dialyse (4) comprenant un canal d'approvisionnement en dialysat (23) connecté à une entrée de la chambre de dialysat (2D) dans le dialyseur (2) et un canal de collecte de dialysat (24) connecté à une sortie de la chambre de dialysat (2D), et
des moyens d'introduction de gaz (32) pour introduire un gaz dans la chambre de dialysat (2D) dans le dialyseur (2) afin de déplacer un dialysat dans une zone dans la chambre de dialysat (2D) autre qu'une zone contenant du gaz,
**caractérisé en ce que** l'appareil comprend en outre des moyens de filtration à contre-courant (27) prévus dans le circuit de dialysat (4) pour faire passer le dialysat de la zone contenant le dialysat dans la chambre de dialysat (2D) dans le circuit de sang (3) via la chambre de sang (2C) dans le dialyseur (2),
et **en ce que** les moyens d'introduction de gaz (32) comprennent un canal ouvert (35) qui est prévu dans le canal d'approvisionnement de dialysat (23) et permet à de l'air d'être introduit dans le canal d'approvisionnement de dialysat (23), et un canal de gaz (38) qui permet une communication fluidique entre le canal ouvert (35) et le canal de collecte de dialysat (24).

2. Dispositif de dialyse (1) selon la revendication 1, **caractérisé en ce que** le dialyseur (2) est orienté verticalement afin que la sortie de la chambre de dialysat (2D) soit positionnée sur une face supérieure, et les moyens d'introduction de gaz (32) sont adaptés pour introduire le gaz dans la chambre de dialysat (2D) dans le dialyseur (2) à la sortie de la chambre de dialysat (2D).
